Europäisches Patentamt

European Patent Office

Office européen des brevets

(11) Veröffentlichungsnummer: **0 517 012 A1**

## EUROPÄISCHE PATENTANMELDUNG

(21) Anmeldenummer: **92108047.9**

(51) Int. Cl.5: **G01N 33/18**

(22) Anmeldetag: **13.05.92**

(30) Priorität: **27.05.91 DE 4117294**

(43) Veröffentlichungstag der Anmeldung:
**09.12.92 Patentblatt 92/50**

(84) Benannte Vertragsstaaten:
**AT DE FR GB IT**

(71) Anmelder: **GRUNDIG E.M.V.
Elektro-Mechanische Versuchsanstalt Max
Grundig holländ. Stiftung & Co. KG.
Kurgartenstrasse 37
W-8510 Fürth/Bay.(DE)**

(72) Erfinder: **Hallas, Ernst, Grundig E.M.V. Max
Grundig
holländ, Stiftung & Co. KG, Kurgartenstrasse
37
W-8510 Fuerth(DE)**

(74) Vertreter: **Eichstädt, Alfred
Grundig E.M.V. Elektro-Mechanische
Versuchsanstalt Kurgartenstrasse 37
W-8510 Fürth(DE)**

(54) **Verfahren und Anordnung zur detektion und Identifikation von Fremdstoffen auf Wasseroberflächen.**

(57) Die Anmeldung betrifft ein Verfahren und eine Anordnung zur Detektion und Identifikation von Fremdstoffen auf Wasseroberflächen durch Bestimmung der Transmissionseigenschaften einer Wasseroberfläche und der durch Wasserdampf entstehenden Gasphase über der Wasseroberfläche.

Aufgabe der Anmeldung ist es daher ein Verfahren und eine Anordnung anzugeben, bei dem eine ständige Überwachung einer Wasseroberfläche möglich ist, wobei die Auslösung eines Fehlalarmes durch Identifikation der Störung vermieden werden soll.

Diese Aufgabe wird gelöst, indem die Transmissionseigenschaften der Wasseroberfläche durch die Abnahme der Lichtintensität eines mehrfach zwischen zwei Spiegeln reflektierten, schräg zur Wasseroberfläche einfallenden Lichtstrahles gemessen wird und gleichzeitig die Transmissionseigenschaft der über der Wasseroberfläche befindlichen und hauptsächlich aus Wasserdampf bestehenden Gasphase durch die Änderung der Intensität eines parallel zur Wasseroberfläche verlaufenden Lichtstrahles bestimmt wird.

Figur 1

EP 0 517 012 A1

Die Erfindung betrifft ein Verfahren und eine Anordnung zur Durchführung des Verfahrens zur Detektion und Identifikation von Fremdstoffen auf Wasseroberflächen durch Bestimmung der Transmissionseigenschaften einer Wasseroberfläche.

Insbesondere Gewässer, beispielsweise Flüsse, in der Nähe von Industrie- oder Kraftwerksanlagen, in die Abwässer dieser Anlagen eingeleitet werden, müssen regelmäßig oder ständig auf Fremdstoffe untersucht werden. Besonders die Öldetektion spielt hierbei eine große Rolle, da eine Verschmutzung mit Öl weitreichende negative Folgen hat.

Zur Erkennung von Verschmutzungen des Wassers durch Öl oder sonstige Fremdstoffe werden häufig Trübungsmeßgeräte eingesetzt. Dazu werden dem Gewässer Proben entnommen, von denen die Trübungswerte gemessen werden, die auf den Grad der Verschmutzung schließen lassen. Die Art der Verschmutzung kann jedoch mit dieser Methode nicht ermittelt werden, da beispielsweise durch Öl verursachte Verschmutzung die gleichen Trübungswerte liefern kann, wie eine durch aufgewühlten Schlamm verursachte Verschmutzung.

Ein Anzeigegerät zum Nachweis der Verschmutzung durch Öl oder ähnliche Verunreinigungen ist in der deutschen Offenlegungsschrift DE 2224310 A1 beschrieben. Dort werden ein oder mehrere Grenzflächen mit einer zu überwachenden Flüssigkeit auf der einen Seite benetzt, während auf der anderen Seite, in einem anderen Medium, ein Strahlenbündel einfällt, dessen Einfallswinkel so gewählt wird, daß er nahe am Grenzwinkel zur Totalreflexion liegt. Bei Vorhandensein von Öl in der zu untersuchenden Flüssigkeit wird der Grenzwinkel überschritten und es findet Totalreflexion statt, so daß eine Signalvorrichtung vom reflektierenden Strahlenbündel betätigt wird.
Bei einem derartigen Anzeigegerät besteht aber der Nachteil, daß jeweils nur eine Probe entnommen wird, die nicht zwingend eine allgemeingültige Aussage über den momentanen Zustand des Gewässers zuläßt.

In der deutschen Offenlegungsschrift DE 3038107 A1 ist ein Verfahren zur berührungsfreien Ermittlung von Öl auf einer Wasseroberfläche beschrieben. Die zu untersuchende Stelle der Wasseroberfläche wird mit einer ultravioletten Lichtquelle bestrahlt, die die Fluoreszenz der erwarteten Ölgruppe anregen soll. Das durch die Fluoreszenzwirkung abgestrahlte Licht wird fotoelektrisch empfangen, wobei die von der Oberfläche reflektierte, durch das UV-Licht der ultravioletten Lichtquelle im wesentlichen sperrende Filter geführte Strahlung, sowie die von der Fluoreszenz herrührende Oberflächenstrahlung vom fotoelektrischen Empfänger aufgenommen werden und die Gesamtintensität der ungerichteten Fluoreszenzstrahlung erfaßt wird.

Der Nachteil dieses Verfahrens liegt in der punktuellen Messung, bei der nicht eine großflächige Oberfläche überprüft wird, sondern nur ein relativ kleiner Bereich.

Es sind weiterhin Verfahren bekannt, bei denen ein Lichtsender unter einem bestimmten Winkel auf die Wasseroberfläche einstrahlt und an anderer Stelle ein Photodetektor angebracht ist, der das reflektierte Licht empfängt. Bei Verschmutzungen der Wasseroberfläche wird der einfallende Lichtstrahl ganz oder teilweise absorbiert, beziehungsweise in geänderter Intensität reflektiert, so daß bei einer Änderung der Intensität des detektierten Lichtstrahls eine Verschmutzung erkannt wird. Der Nachteil solcher Verfahren liegt wieder in der punktuellen Messung, die nur begrenzt einen Schluß auf die gesamte Wasseroberfläche zuläßt. Weiterhin können leicht Störungen durch Objekte, die auf der Wasseroberfläche schwimmen, auftreten.

Aufgabe der Erfindung ist es daher, ein Verfahren und eine Anordnung zur Durchführung des Verfahrens anzugeben, bei dem eine ständige Überwachung einer Wasseroberfläche möglich ist und die Überwachung nicht auf einzelne Proben oder Stellen beschränkt ist, wobei die Auslösung eines Fehlalarmes durch Identifikation der Störung vermieden werden soll.

Diese Aufgabe wird durch das erfindungsgemäße Verfahren und die Anordnung zur Durchführung des Verfahrens gelöst, indem die Transmissionseigenschaften der Wassseroberfläche durch die Abnahme der Lichtintensität eines mehrfach zwischen zwei Spiegeln reflektierten, schräg zur Wasseroberfläche einfallenden Lichtstrahles, gemessen wird und gleichzeitig die Transmissionseigenschaft der über der Wasseroberfläche befindlichen und hauptsächlich aus Wasserdampf bestehenden Gasphase durch die Änderung der Intensität eines parallel zur Wasseroberfläche verlaufenden Lichtstrahles bestimmt wird.
Durch das erfindungsgemäße Verfahren wird das Gewässer in zweifacher Weise überwacht. Zum ersten wird die Gasphase über der Wasseroberfläche durch einen parallel zur Wasseroberfläche verlaufenden Lichtstrahl bezüglich der Transmissionseigenschaft ausgewertet. Das von einem Lichtsender mit konstanter Intensität abgestrahlte Licht wird von einem Detektor empfangen. Durch den über der Wasseroberfläche befindlichen Wasserdampf wird der Lichtstrahl abgeschwächt. Fremdstoffe auf der Wasseroberfläche verändern die Transmissionseigenschaften des Wasserdampfes in der Weise, daß bestimmte Gaskomponenten den Lichtstrahl stärker oder weniger stark dämpfen, oder daß beispielsweise die Wasserdampfbildung bei Öl auf der Wasseroberfläche stark eingeschränkt bzw. unterbunden wird, was eine Erhöhung der Transmission zur Folge hat. Die Fremdstoffe verändern also die Intensität des empfangenen Lichtstrahles und können dadurch erkannt werden.

Weiterhin wird ein zweiter Lichtstrahl schräg zur Wasseroberfläche eingestrahlt und von einem sich nahe unter der Wasseroberfläche befindlichen Spiegel reflektiert. Ein zweiter Spiegel befindet sich über der Wasseroberfläche und reflektiert den bereits am ersten Spiegel reflektierten Lichtstrahl wieder und so fort. Ein zweiter Detektor empfängt den mehrfach reflektierten Lichtstrahl. Befinden sich Fremstoffe auf der Wasseroberfläche, dann wird der Lichtstrahl absorbiert oder zumindest in seiner Intensität geändert, so daß sich die Intensität des im Detektor empfangenen Lichtstrahles ändert und ein Fremdstoff erkannt wird. Die Detektoren, die den mehrfach reflektierten und den parallel zur Wasseroberfläche verlaufenden Lichtstrahl empfangen, sind zur Auswertung der Meßergebnisse mit einer Auswerteeinheit verbunden.

Der Vorteil des erfindungsgemäßen Verfahrens liegt neben der Überwachung einer gesamten Wasseroberfläche, im Gegensatz zur punktuellen Überwachung, in der Möglichkeit zur Identifizierung von Störkörpern, die nicht zur Alarmauslösung führen sollen, da sie keine umweltbelastende Störung darstellen. So kann beispielsweise ein größeres, auf der Wasseroberfläche treibendes Holzstück erkannt werden, da in diesem Fall der parallel zur Wasseroberfläche verlaufende Lichtstrahl ganz unterbrochen wird und ebenso der mehrfach reflektierte Lichtstrahl unterbrochen wird.

In einer vorteilhaften Ausgestaltung der Erfindung werden die Frequenzen der Lichtsender so eingestellt, daß bei Vorliegen eines zu detektierenden Fremdstoffes die Änderung des Transmissionsverhaltens im Vergleich zum Normalfall maximal wird. Bei Untersuchung der Wasseroberfläche auf Öl wird demgemäß die Frequenz des Lichtsenders, der schräg zur Wasseroberfläche einstrahlt, so gewählt, daß der Lichtstrahl durch Öl absorbiert wird.

Im entprechenden Empfänger wird im Störungsfall kein oder nur ein schwaches Lichtsignal empfangen. Die Frequenz des parallel zur Wasseroberfläche verlaufenden Lichtstrahls wird so gewählt, daß der Lichtstrahl durch den Wasserdampf stark gedämpft wird. Im Störungsfall, d.h. Öl auf der Wasseroberfläche, verringert sich die Absorption, da das Öl die Bildung von Wasserdampf einschränkt und die Intensität des im entsprechenden Detektor empfangenen Lichtstrahls zunimmt.

In einer vorteilhaften Ausgestaltung der erfindungsgemäßen Anordnung wird die gesamte Meßvorrichtung schwimmend auf der Wasseroberfläche angeordnet. Zu diesem Zweck sind zwei schwimmfähige Elemente vorgesehen, an deren Unterseite ein Spiegel derart angebracht ist, daß er die beiden schwimmfähigen Elemente verbindet und in festem Abstand zueinander hält. Die schwimmfähigen Elemente tauchen leicht in das Wasser ein, wobei der untere Spiegel unter der Wasseroberfläche liegt.

Eine weitere vorteilhafte Ausgestaltung der Anordnung ergibt sich, wenn die Meßvorrichtung mit einer Haube abgedeckt wird, die an den schwimmfähigen Elementen angebracht ist, und die Lichtsender und Detektoren an den schwimmfähigen Elementen bzw. an der Haube angebracht sind.

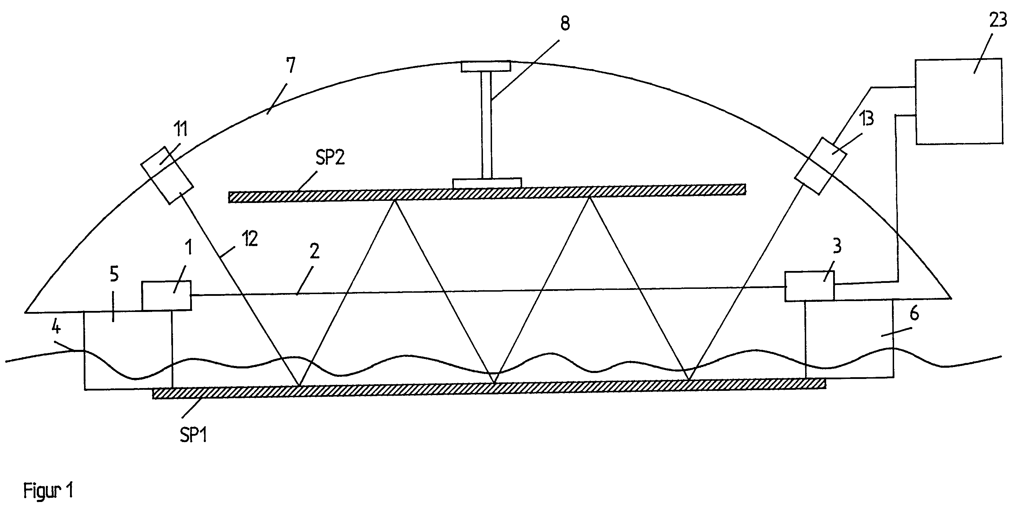

Im folgenden wird die erfindungsgemäße Anordnung an Hand eines Ausführungsbeispieles nach Figur 1 erläutert.

Diese Anordnung besteht aus den schwimmfähigen Elementen 5 und 6, an denen die Meßeinrichtung angebracht ist. Der Lichtsender 1, der einen Lichtstrahl 2 parallel zur Wasseroberfläche abstrahlt, ist auf dem schwimmfähigen Element 5 aufgebracht. Der diesen Lichtstrahl 2 empfangende Photodetektor 3 ist auf dem ebenfalls schwimmfähigen Element 6 angeordnet. An der Unterseite der Elemente 5 und 6 ist der erste Spiegel SP1 angebracht, der sich unter der Wasseroberfläche 4 befindet. Die Haube 7 ist ebenfalls an den schwimmfähigen Elementen 5 und 6 befestigt. An der Haube sind der Lichtsender 11 und auf der gegenüberliegenden Seite der Detektor 13 angebracht. Der Lichtsender 11 strahlt einen Lichtstrahl 12 unter einem vorgegebenen Winkel schräg auf die Wasseroberfläche und damit auf den unter der Wasseroberfläche liegenden Spiegel SP1, der den Lichtstrahl reflektiert, so daß er auf den Spiegel SP2 trifft und von diesem wieder reflektiert wird. Nach mehrfacher Reflektion trifft der Lichtstrahl 12 auf den Photodtektor 13. Dieser ist, ebenso wie der Detektor 3, mit einer Auswerteeinheit 23, deren Wirkungsweise später beschrieben wird, verbunden. Der Spiegel SP2 ist durch ein Verbindungsstück 8 an der Haube 7 befestigt.

Durch die schwimmfähige Anordnung wird eine wasserstandsgeregelte Nachführung der Meßvorrichtung unnötig und die Anordnung ist dadurch flexibel einsetzbar. Die Sender und die Detektoren können aber auch an den gegenüberliegenden Ufern eines Gewässers angebracht werden, wobei in diesem Fall eine wasserstandsgeregelte Nachführung sowohl für die Sender und Detektoren als auch für die Spiegel notwendig ist.

Vom Sender 1 wird ein Lichtstrahl 2 parallel zur Wasseroberfläche ausgesendet, der vom Detektor 3 empfangen wird. Mit diesem Lichtstrahl 2, dessen Wellenlänge vorteilhafterweise bei etwa 1,9 $\mu m$ liegt, so daß er vorwiegend durch Wasserdampf absorbiert wird, werden Veränderungen der Transmissionseigenschaften der Gasphase über der Wasseroberfläche sowie größere Objekte auf der Wasseroberfläche detektiert.

Vom Sender 11 wird ein Lichtstrahl 12 schräg zur Wasseroberfläche 4 ausgesendet, der nach mehrfachen Reflektionen an den Spiegeln SP1 und SP2 vom Detektor 13 empfangen wird. Die Wellenlänge des Lichtstrahls 12 liegt vorteilhafterweise bei etwa 3,5 $\mu$m, so daß er vorwiegend durch Öl absorbiert wird. Mit dieser zweiten Sender-/Detektoranordnung 11,13 werden also hauptsächlich Fremdstoffe auf der Wasseroberfläche durch direkte Absorption des Lichtes vom Fremdstoff erfaßt.

Die von den Detektoren 3 und 13 empfangenen Signale werden in der Auswerteeinheit 23 verarbeitet. Zur Verdeutlichung der Arbeitsweise und der Auswertekriterien der Auswerteeinheit 23 werden verschiedene mögliche Fälle betrachtet.

Im Normalzustand wird im Detektor 3 ein durch Wasserdampf abgeschwächter Lichtstrahl empfangen, während im Detektor 13 der an den Spiegeln SP1 und SP2 mehrfach reflektierte Lichtstrahl - unter Berücksichtigung der normalen Reflektionsverluste und der durch Wasserdampf entstehenden Absorptionsverluste - empfangen wird. Ausgehend von diesem Normalzustand sind folgende Störzustände denkbar:

- Erhöhung der Intensität des im Detektor 3 empfangenen Lichtstrahls 2 und Verringerung der Intensität des im Empfänger 13 empfangenen Lichtstrahls 12. Die Ursache ist ein Fremdstoff auf der Wasseroberfläche 4, der den Lichtstrahl 12 absorbiert und die Wasserdampfbildung einschränkt. In diesem Fall wird von der Auswerteeinheit 23 Öl auf der Wasseroberfläche erkannt und Alarm ausgelöst.
- Keine Veränderung des im Detektor 3 empfangenen Lichtstrahls und Unterbrechung des Lichtstrahls 12. Die Ursache ist beispielsweise ein Laubblatt auf der Wasseroberfläche 4, das den Lichtstrahl 12 unterbricht, aber die Wasserdampfbildung nicht beeinträchtigt, da das Blatt benetzt wird. In diesem Fall wird kein Alarm von der Auswerteeinheit 23 ausgelöst.
- Unterbrechung beider Lichtstrahlen. Die Ursache ist ein größeres Störobjekt, wie beispielsweise ein Ast, der sowohl den parallel zur Wasseroberfläche verlaufenden Lichtstrahl 2 als auch den mehrfach reflektierten Lichtstrahl 12 unterbricht. In diesem Fall wird kein Alarm ausgelöst, da die Ursache kein künstlich erzeugter, umweltbelastender Störfall ist.

Die oben beschriebenen Fälle sind beispielhaft und ohne Einschränkung der Allgemeinheit ausgewählt und beziehen sich vorwiegend auf die Detektion und Identifikation von Öl auf Wasseroberflächen. Sie zeigen eine Möglichkeit der Auswertekriterien der Auswerteeinheit 23 auf. In anderen Anwendungsfällen kann die Auswertung unter anderen Gesichtspunkten erfolgen.

**Patentansprüche**

1. Verfahren zur Detektion und Identifikation von Fremdstoffen auf Wasseroberflächen durch Bestimmung der Transmissionseigenschaft einer Wasseroberfläche **dadurch gekennzeichnet,** daß die Transmissionseigenschaft der Wasseroberfläche durch die Abnahme der Lichtintensität eines mehrfach zwischen zwei Spiegeln reflektierten, schräg zur Wasseroberfläche einfallenden Lichtstrahls gemessen wird, und daß weiterhin die Transmissionseigenschaft der Gasphase durch die Änderung eines parallel zur Wasseroberfläche verlaufenden Lichtstrahles bestimmt wird.

2. Vefahren nach Anspruch 1, **dadurch gekennzeichnet,** daß die Frequenzen der Lichtstrahlen so gewählt werden, daß bei Vorliegen der festzustellenden Fremdstoffe eine maximale Änderung der Transmissionseigenschaften vorliegt.

3. Anordnung zur Durchführung des Verfahrens nach Anspruch 1 oder 2, **dadurch gekennzeichnet,** daß die Anordnung
   - einen ersten Lichtsender (1) enthält, der einen Lichtstrahl (2) parallel zur Wasseroberfläche (4) in Richtung eines gegenüberliegenden Detektors (3) strahlt,
   - einen ersten Spiegel (SP1) und einen zweiten Spiegel (SP2) enthält, von denen der erste über der Wasseroberfläche (4) und der zweite unter der Wasseroberfläche (4) angeordnet ist,
   - einen zweiten Lichtsender (11) enthält, der einen Lichtstrahl (12) unter einem vorgebbaren Winkel auf den ersten Spiegel (SP1) einstrahlt, so daß der Lichtstrahl (12) mehrfach durch den ersten und den zweiten Spiegel reflektiert wird und von einem zweiten Detektor (13) empfangen wird,
   - eine Auswerteeinheit (23) enthält, mit der die von den Detektoren (3,13) empfangenen Lichtsignale ausgewertet werden.

4. Anordnung nach Anspruch 3, **dadurch gekennzeichnet,** daß die Anordnung schwimmend auf der Wasseroberfläche liegt, wobei der Spiegel (SP1) an der Unterseite von schwimmfähigen Elementen (5,6) derart angebracht ist, daß er diese Elemente verbindet und in einem konstanten Abstand hält.

5. Anordnung nach Anspruch 3 oder 4, **dadurch**

**gekennzeichnet,** daß die Anordnung mit einer Haube (7) abgedeckt ist, die an den schwimmfähigen Elementen (5,6) angebracht ist.

6. Anordnung nach einem oder mehreren der Ansprüche 3 bis 5, **dadurch gekennzeichnet,** daß der Lichtsender (1) und der Detektor (3) auf den schwimmfähigen Elementen (5) und (6) angebracht sind.

7. Anordnung nach einem oder mehreren der Ansprüche 3 bis 6, **dadurch gekennzeichnet,** daß der Lichtsender (11) und der Detektor (13) an der Haube (7) angebracht sind.

Figur 1

EP 0 517 012 A1

Europäisches Patentamt

# EUROPÄISCHER RECHERCHENBERICHT

Nummer der Anmeldung

EP 92 10 8047

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-2 354 125 (SIEMENS AG) <br> * Seite 2; Abbildung 1 * <br> --- | 1,3 | G01N33/18 |
| A | US-A-3 364 351 (T. Y. PALMER ET AL.) <br> * Spalte 1, Zeile 15 - Zeile 25; Abbildung 1 * <br> --- | 1,3 | |
| A | DE-A-2 847 689 (BASF AG) <br> * Anspruch 1; Abbildung 1 * <br> --- | 3 | |
| A | DE-U-1 944 991 (BRAN & LÜBBE) <br> * Abbildung 1 * <br> --- | 3 | |
| A | DE-A-2 657 851 (BROWN BOVERI & CO AG) <br> * Ansprüche 1-5; Abbildung 1 * <br> --- | 3 | |
| A | DE-A-2 164 670 (BROWN BOVERI & CO AG) <br> * Abbildung 1 * <br> ----- | 1,3 | |

**RECHERCHIERTE SACHGEBIETE (Int. Cl.5)**

G01N

Der vorliegende Recherchenbericht wurde für alle Patentansprüche erstellt

| Recherchenort | Abschlußdatum der Recherche | Prüfer |
|---|---|---|
| BERLIN | 08 SEPTEMBER 1992 | BRISON O.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

.......................................................................................
& : Mitglied der gleichen Patentfamilie, übereinstimmendes Dokument

EPO FORM 1503 03.82 (P0403)